(19)

Europäisches Patentamt
European Patent Office
Office européen des brevets

(11) **EP 4 582 454 A1**

(12) **EUROPEAN PATENT APPLICATION**
published in accordance with Art. 153(4) EPC

(43) Date of publication:
**09.07.2025 Bulletin 2025/28**

(21) Application number: 23859107.7

(22) Date of filing: **09.08.2023**

(51) International Patent Classification (IPC):
**C07K 19/00** (2006.01)    **C12N 15/867** (2006.01)
**C12N 5/10** (2006.01)

(52) Cooperative Patent Classification (CPC):
**A61K 35/17; A61K 38/20; A61K 48/00;
A61P 35/00; C07K 19/00; C12N 5/10; C12N 15/62;
C12N 15/867**

(86) International application number:
**PCT/CN2023/111999**

(87) International publication number:
**WO 2024/046072 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.08.2022 CN 202211042674**

(71) Applicant: **Neukio Biotherapeutics (Shangai ) Co.,
Ltd.
Shanghai 200131 (CN)**

(72) Inventors:
• **WANG, Yuan**
  **Shanghai 200131 (CN)**
• **ZHANG, Lei**
  **Shanghai 200131 (CN)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **IMMUNE CELL HAVING MEMBRANE-BOUND IL-21, AND PREPARATION METHOD THEREFOR AND USE THEREOF**

(57)     Disclosed are an immune cell having membrane-bound IL-21 (mbIL-21), and a preparation method therefor and a use thereof. Specifically, disclosed are mbIL-21 and a use thereof in immunotherapy. mbIL-21 can enhance the expansion capability and killing power of specific immune cells, thereby enhancing the effect of NK cell therapy.

## Description

### Technical field

The present invention relates to the field of cell therapy, specifically to an immune cell having membrane-bound IL-21, and a preparation method therefor and a use thereof.

### Background

The homeostasis of the immune system relies on two major components: innate and adaptive immune responses, both of which are regulated by a series of cytokines. The cytokine receptor γ-chain (γc) family is one of the most extensively studied groups of cytokines, including IL-2, IL-4, IL-7, IL-9, IL-15, and IL-21. This group of cytokines exhibits broad pleiotropic effects, regulating both innate and adaptive immune systems, collectively promoting the development of various immune cell populations, modulating cell differentiation, and promoting survival or inducing apoptosis depending on the cellular environment.

In cell therapy, NK cell therapy is a promising anti-cancer strategy. However, the widespread clinical success of NK cell therapy is to some extent limited by the challenge of the manufacture of large doses of NK cells, which may be necessary for clinical efficacy.

Moreover, studies have shown that compared to T cells, the persistence of NK cells in the host body is relatively short, which may affect the tumor-killing efficacy of NK cells. Treatment in combination with IL-2 can to some extent prolong the persistence of NK cells in the host body, but IL-2 may also lead to the massive proliferation of Treg cells that exert immunosuppressive effects.

Therefore, there is an urgent need in this field to develop NK cells with longer *in vivo* persistence and higher efficacy.

### Summary of the invention

The purpose of the present invention is to provide NK cells with longer *in vivo* persistence and higher efficacy, as well as a preparation method therefor and a use thereof.

In the first aspect of the present invention, it provides a recombinant protein, wherein the recombinant protein is a membrane-bound IL-21 (mbIL-21), and the recombinant protein has the following characteristics:

(a) the recombinant protein has the structure shown in Formula I:

$$Z1\text{-}Z2\text{-}Z3\text{-}Z4 \qquad (I)$$

wherein,

Z1 is a signal peptide or absent;
Z2 is an IL-21 polypeptide;
Z3 is a CD8 hinge region;
Z4 is a CD8 transmembrane region;
"-" represents a peptide bond or a linker peptide;

(b) the recombinant protein has a sequence shown in positions 1-249 or 25-249 of SEQ ID No: 1; or has a sequence shown in positions 1-246 or 22-246 of SEQ ID No: 2.

In another preferred embodiment, Z2 comprises a wild-type or mutant IL-21 element.
In another preferred embodiment, Z2 comprises IL-21 elements from human and non-human mammals.
In another preferred embodiment, Z3 and Z4 are each independently wild-type or mutant.
In another preferred embodiment, Z3 and Z4 are each independently derived from human and non-human mammals.
In another preferred embodiment, Z1 comprises the following sequences:

| IL-21SP | MRSSPGNMERIVICLMVIFLGTLV | SEQ ID No: 3 |
|---------|---------------------------|--------------|
| CD8αSP  | MALPVTALLLPLALLLHAARP    | SEQ ID No: 4 |

(continued)

| CSF2SP | MWLQSLLLLGTVACSIS | SEQ ID No: 5 |
|---|---|---|
| DAP12SP | MGGLEPCSRLLLLPLLLAVSG | SEQ ID No: 6 |
| CD16SP | MWQLLLPTALLLLVSA | SEQ ID No: 7 |
| CD56SP | MLQTKDLIWTLFFLGTAVS | SEQ ID No: 8 |
| IL-8SP | MTSKLAVALLAAFLISAALC | SEQ ID No: 9 |

In the second aspect of the present invention, it provides an isolated polynucleotide encoding the recombinant protein of the first aspect of the present invention.

In the third aspect of the present invention, it provides a vector comprising the polynucleotide of the second aspect of the present invention.

In another preferred embodiment, the vector comprises a plasmid or a viral vector.

In another preferred embodiment, the vector is selected from the group consisting of: a retroviral vector, an adenoviral vector, a lentiviral vector, and an AAV vector.

In the fourth aspect of the present invention, it provides a genetically engineered cell, wherein the genetically engineered cell is selected from the group consisting of: an embryonic stem cell, a mesenchymal stem cell, an iPSC cell (induced pluripotent stem cell), an NK cell, a T cell, a lymphocyte, and a combination thereof.

In another preferred embodiment, the genetically engineered cell comprises an NK cell, a T cell, or a lymphocyte.

Further more, the genetically engineered cell has following the characteristics:

(a) comprising the vector of the third aspect of the present invention; or
(b) having the polynucleotide of the second aspect of the present invention integrated into its genome.

In another preferred embodiment, the genetically engineered cell expresses membrane-bound IL-21 (mbIL-21), and has the mbIL-21 protein being present on the cell membrane.

In the fifth aspect of the present invention, it provides a pharmaceutical composition, which comprises the genetically engineered cell of the fourth aspect of the present invention and a pharmaceutically acceptable carrier.

In another preferred embodiment, the genetically engineered cell is an NK cell, a T cell, or a lymphocyte.

In another preferred embodiment, the pharmaceutical composition is for use in cell therapy.

In another preferred embodiment, the cell therapy is for use in the treatment of tumors.

In the sixth aspect of the present invention, it provides a use of the genetically engineered cell of the fourth aspect of the present invention in the manufacture of a medicament for immunotherapy.

In the seventh aspect of the present invention, it provides a cellular immunotherapy, which comprises a step of: administering the genetically engineered cell of the fourth aspect of the present invention to a patient, wherein the genetically engineered cell comprises the vector of the third aspect of the present invention, or has the polynucleotide of the second aspect of the present invention integrated into its genome.

In another preferred embodiment, the genetically engineered cell expresses membrane-bound IL-21 (mbIL-21), and has the mbIL-21 protein being present on the cell membrane.

In another preferred embodiment, the genetically engineered cell is selected from the group consisting of: an embryonic stem cell, a mesenchymal stem cell, an iPSC cell, an NK cell, a T cell, a lymphocyte, and a combination thereof.

In another preferred embodiment, the patient is a tumor patient, preferably with a tumor selected from the group consisting of: hematological system tumor, lung cancer, liver cancer, neuroblastoma, ovarian tumor, rhabdomyosarcoma, breast cancer, gastric cancer, gastrointestinal tumor, renal cancer, and prostate cancer.

In another preferred embodiment, the patient is a tumor patient, preferably an early-stage tumor patient who does not require radiotherapy or chemotherapy, or a late-stage tumor patient who cannot undergo radiotherapy or chemotherapy.

In another preferred embodiment, the immunotherapy is administered by intravenous injection or local tissue injection.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

## Description of the drawings

Figure 1 shows the structural diagram of the recombinant protein mbIL-21.
Figure 2 shows the isolation, culture, and identification of NK cells.
Figure 3 shows the detection of mbIL-21 expression on the surface of NK cells.
Figure 4 shows that mbIL-21 promotes the division and proliferation of NK cells.
Figure 5 shows that mbIL-21 stimulates the proliferation of NK cells.
Figure 6 shows that mbIL-21 promotes the phosphorylation of STAT3 in NK cells.
Figure 7 shows the effect of mbIL-21 on the expression of CD226.

## Detailed description

After extensive and in-depth research, the inventors unexpectedly discovered that a recombinant protein with a special structure, namely membrane-bound IL-21 (mbIL-21), is expressed in a manner fixed on the cell membrane surface of lymphocytes such as NK cells, thereby significantly promoting the division and proliferation of NK cells, extending the *in vivo* persistence of NK cells, and significantly reducing or eliminating the potential risks associated with the secretion of IL-21 that may be detrimental to NK cell therapy, finally enhancing the efficacy of NK cell immunotherapy. On this basis, the present invention is completed.

### Terms

To facilitate a better understanding of the present disclosure, certain terms are first defined. As used in the present application, unless otherwise expressly defined herein, each of the following terms shall have the meaning given below.

The term "about" may refer to a value or composition within an acceptable error range of a particular value or composition determined by a person of ordinary skill in the art, which will partly depend on how the value or composition is measured or determined.

The term "administration" refers to the physical introduction of the product of the present invention into a subject using any one of the various methods and delivery systems known to those skilled in the art, including intravenous, intratumoral, intramuscular, subcutaneous, intraperitoneal, intraspinal, or other parenteral routes of administration, such as by injection or infusion.

### Membrane-bound IL-21

As used herein, the terms "the protein of the present invention", "membrane-bound IL-21", "membrane-bound IL-21 protein", "the membrane-bound IL-21 of the present invention", "mbIL-21 protein" and the like can be used interchangeably to refer to the recombinant protein of the first aspect of the present invention.

Typically, the membrane-bound IL-21 protein of the present invention has the structure shown in Formula I.

### NK cell(s)

As used herein, the terms "NK cell(s)", "memory NK cell(s)", "natural killer cell(s)", "natural killer (NK) cell(s)", "NK", and the like can be used interchangeably to refer to a major class of immune effector cells that protect the body from viral infections and tumor cell invasion through non-antigen-specific pathways. NK cells that have been engineered (genetically modified) may acquire new functions, including the ability to specifically recognize tumor antigens and enhanced cytotoxic activity against tumor cells.

### Vector

The present invention provides a vector comprising the nucleotide of the present invention. Vectors derived from retroviruses, such as lentiviruses, are suitable tools for achieving long-term gene transfer, as they allow the long-term and stable integration of transgenes into the cellular genome and enable the transgenes to replicate along with the replication of the progeny cell genome. Lentiviral vectors have advantages over vectors derived from oncogenic retroviruses, such as murine leukemia virus, because they can transduce non-dividing cells and have the benefit of low immunogenicity.

## Genetically engineered cell(s) expressing membrane-bound IL-21

The present invention provides a genetically engineered cell expressing the membrane-bound IL-21 of the present invention, wherein the genetically engineered cell is selected from the group consisting of: an embryonic stem cell, a mesenchymal stem cell, an iPSC cell (induced pluripotent stem cell), an NK cell, a T cell, a lymphocyte, and a combination thereof.

## Pharmaceutical composition

The present invention provides a pharmaceutical composition comprising the engineered immune cell of the present invention, and a pharmaceutically acceptable carrier, diluent, or excipient.

In one embodiment, the formulation is a liquid formulation. Preferably, the formulation is an injection.

In one embodiment, the formulation may include buffers such as neutral buffered saline, sulfate-buffered saline, etc.; carbohydrates such as glucose, mannose, sucrose, dextran, or mannitol; proteins; polypeptides or amino acids such as glycine; antioxidants; chelating agents such as EDTA or glutathione; and preservatives. The formulation of the present invention is preferably formulated for intravenous administration or local tissue administration.

## Therapeutic application

The present invention comprises therapeutic applications involving cells (e.g., NK cells) transduced with vectors comprising the recombinant protein of the present invention (such as lentiviral vectors). NK cells can target surface markers on tumor cells, and the proliferation efficiency of the transduced NK cells, as well as their cytotoxicity against tumor cells, are significantly enhanced.

The main advantages of the invention include:

(a) The mbIL-21 of the present invention can significantly enhance the proliferation capability and cytotoxicity of NK cells both *in vivo* and *in vitro,* thereby enhancing the efficacy of NK cell therapy.

(b) The mbIL-21 nucleotide fragment of the invention can be inserted into the expressible sites of pluripotent stem cells (such as iPSCs, ESCs, etc.) through genetic editing. The immune cells, represented by NK cells, derived from differentiation, possess enhanced proliferative and cytotoxic capabilities.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

## Example 1 Design of the Recombinant Protein mb-IL21

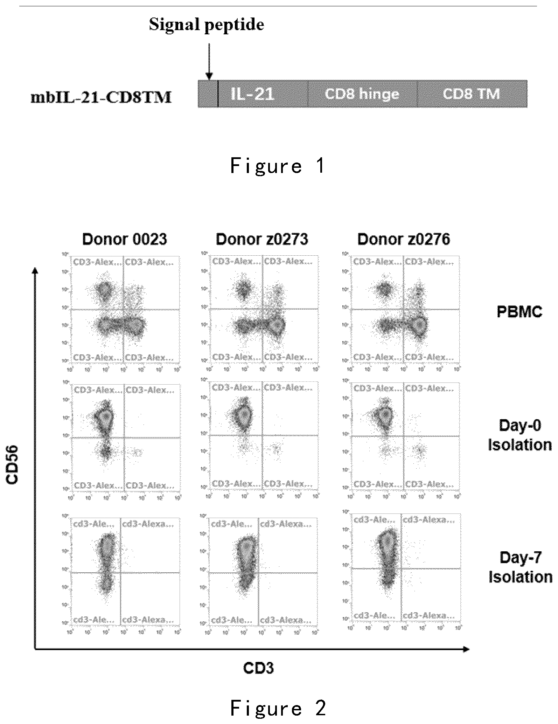

The main structure of the recombinant protein mb-IL21 in this example is shown in Figure 1, wherein the signal peptide sequence of IL21, the hinge region sequence of CD8, and the transmembrane (TM) sequence of CD8 are used.

The amino acid sequence of mbIL-21 in this example is shown in SEQ ID No: 1 or 2.

IL21SP-**IL21**-GGGGS-CD8 hinge-CD8TM

MRSSPGNMERIVICLMVIFLGTLV**HKSSSQGQDRHMIRMRQLIDIVDQLKN YVNDLVPEFLPAPEDVETNCEWSAFSCFQKAQLKSANTGNNERIINVSIK KLKRKPPSTNAGRRQKHRLTCPSCDSYEKKPPKEFLERFKSLLQKMIHQ HLSSRTHGSEDS**GGGGSPAPRPPTPAPTIASQPLSLRPEACRPAAGGAVHTRG LDFACDIYIWAPLAGTCGVLLLSLVITLYCNHRNRRRVCKCPRPVV\*

(SEQ ID No: 1)

CD8aSP-**IL21**-GGGGS-CD8 hinge-CD8TM

MALPVTALLLPLALLLHAARP**HKSSSQGQDRHMIRMRQLIDIVDQLKNYV NDLVPEFLPAPEDVETNCEWSAFSCFQKAQLKSANTGNNERIINVSIKKL KRKPPSTNAGRRQKHRLTCPSCDSYEKKPPKEFLERFKSLLQKMIHQHL SSRTHGSEDS**GGGGSPAPRPPTPAPTIASQPLSLRPEACRPAAGGAVHTRGLD FACDIYIWAPLAGTCGVLLLSLVITLYCNHRNRRRVCKCPRPVV*

(SEQ ID No: 2)

The nucleotide sequence of the recombinant protein mb-IL21 in this example is the artificially synthesized nucleotide sequence corresponding to SEQ ID No: 1 or 2.

In addition, an mb-IL21 containing a P2A sequence was also constructed to connect mbIL-21 with the mCherry reporter gene via P2A, making it easier and faster to monitor the stability of the entire expression system when evaluating and screening the design schemes of these molecules in the early stage.

## Example 2 Isolation, Culture, Expansion, and Identification of NK Cells

NK cells were isolated from human peripheral blood mononuclear cells (PBMC). After culture and expansion, the obtained NK cells were identified.

The identification results are shown in Figure 2. The results indicate that a population of NK cells with a proportion of CD3$^+$CD56$^+$ NK cells greater than 90% can be obtained through isolation and expansion culture.

## Example 3 Preparation of Genetically Engineered NK Cells Expressing mbIL-21

In order to verify the function of mbIL-21, the coding sequence of mbIL-21 was cloned into a retroviral vector, and retroviruses were packaged and used to infect NK cells. Subsequently, the expression of IL-21 on the NK cell membrane was detected by flow cytometry, revealing that the expression ratio of IL-21 on NK cells from different donors ranged from 25% to 40%. In this example, the mbIL-21 used was IL21SP-**IL21**-GGGGS-CD8 hinge-CD8TM (SEQ ID No: 1).

Results

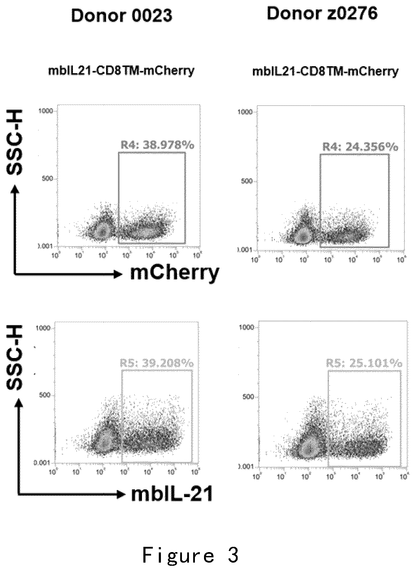

As shown in Figure 3, NK cells expressing mbIL-21 on the cell surface were successfully obtained.

## Example 4 The Effect of mbIL-21 on NK Cell Proliferation

In order to verify the effect of mbIL-21 on NK cell proliferation, in this example, NK-Ctrl and NK-mbIL-21 cells were labeled with CFSE and tested under culture conditions with either the absence of IL-2 (W/O IL-2) or low concentration of IL-2 (20 U/ml IL-2).

The mbIL-21 used in this example was IL21SP-**IL21**-GGGGS-CD8 hinge-CD8TM (SEQ ID No: 1).

The method was as follows: 1. 6E6 NK-Ctrl or NK-mbIL-21 cells were harvested and centrifuged at 300g for 5 minutes at room temperature, after which the supernatant was discarded. 2. After the addition of 10 mL of room-temperature PBS, the cells were gently dispersed by pipetting, and then centrifuged at 300g for 5 minutes at room temperature, with the supernatant being discarded. 3. 6 μL of CellTrace™ CFSE dye was diluted into 6 mL of PBS to prepare a mixture solution. 4. The cells were resuspended in the mixture solution prepared in the previous step at a ratio of 1 mL of mixture solution per 1E6 cells. 5. The resuspended cells were incubated in a 37°C incubator in the dark for 20 minutes to allow staining. 6. Five times the volume of complete medium was added, and the cells were further incubated in a 37°C incubator in the dark for 5 minutes to quench the staining. 7. The cells were centrifuged at 300g for 5 minutes at room temperature, and the supernatant was discarded. 7. The cells were washed 2-3 times with 10 mL of PBS at room-temperature. 8. The cells were resuspended in 12 mL of NK medium and adjusted to a cell density of 5E5/ml, then the cells were inoculated into a 12-well plate, and subjected to culture in a 37°C incubator for 3-5 days before analysis.

Results

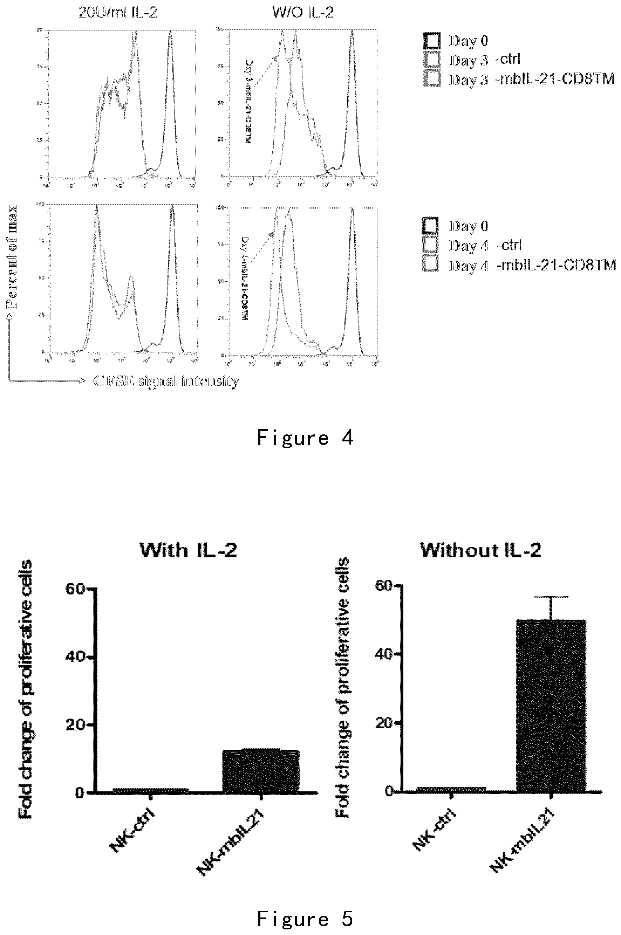

The results are shown in Figures 4 and 5. On Day 0, the CFSE signal intensity of NK-Ctrl and NK-mbIL-21 was consistent. On Day 3 and Day 4, under culture conditions without IL-2, mbIL-21 significantly promoted the division and proliferation of NK cells. In the presence of IL-2, since IL-2 itself can significantly stimulate NK cell proliferation, mbIL-21 did not exert additional effects in this case. After modification with mbIL-21 designed in the present invention, NK cells can proliferate without dependence on exogenous cytokines.

**Example 5 The Effect of mbIL-21 on STAT3 Phosphorylation**

STAT3 plays a major role in the signal transduction and proliferation effects of IL-21. In this example, to investigate the effect of mbIL-21 on STAT3 phosphorylation, the expression of phosphorylated STAT3 (pSTAT3) in NK-Ctrl and NK-mbIL-21 cells was detected by flow cytometry, and the mean fluorescence intensity (MFI) of pSTAT3 was statistically analyzed.

The mbIL-21 used in this example was IL21SP-**IL21**-GGGGS-CD8 hinge-CD8TM.

The method was as follows: 1. The cells were washed twice with 10 mL of BD Pharmingen™ Stain Buffer (Cat. No. 554656). 2. The required amount of BD Phosflow™ Lyse/Fix Buffer (5× concentrate) was diluted with deionized or distilled water at a ratio of 1:5, and the solution was then preheated to 37°C. 3. The cells were evenly dispersed by pipetting with 500 μL of preheated 1× BD Phosflow™ Lyse/Fix Buffer (Cat. No. 558049) and incubated at 37°C for 10 minutes. 4. The cells were washed twice with 1 mL of BD Pharmingen™ Stain Buffer (Cat. No. 554656). 5. Before use, BD Phosflow™ Perm Buffer III was placed at -20°C or 4°C for cooling. 6. The cells were gently resuspended with 500 μL of cooled BD Phosflow™ Perm Buffer III (Cat. No. 558050) and incubated on ice for 30 minutes. 7. The cells were washed twice with 1 mL of BD Pharmingen™ Stain Buffer (Cat. No. 554656). 8. The cells were resuspended in 100 μL of BD Pharmingen™ Stain Buffer (FBS) and stained with Alexa Fluor® 647 Mouse IgG2a Isotype Control (Cat. No. 558053) and Alexa Fluor® 647 Mouse Anti-Stat3 (pY705) antibody (Cat. No. 557815) at room temperature in the dark for 30 minutes. 9. The cells were washed once with BD Pharmingen™ Stain Buffer and resuspended in 200 μL of BD Pharmingen™ Stain Buffer for analysis by flow cytometry.

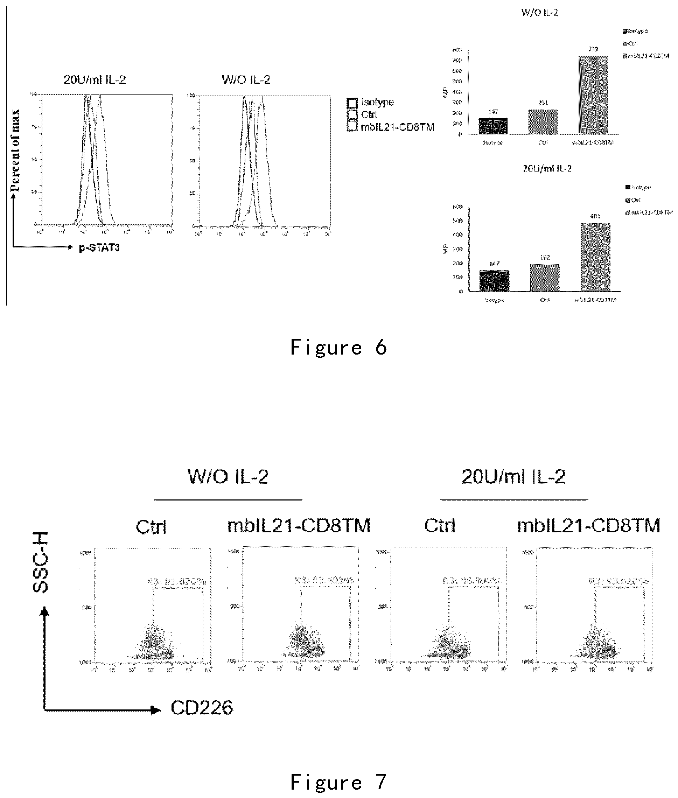

The results are shown in Figure 6, which indicate that the expression of mbIL-21 can significantly promote the phosphorylation of STAT3.

**Example 6 The Effect of mbIL-21 on CD226 Expression**

CD226, also known as DNAM-1, is a member of the immunoglobulin superfamily and is primarily expressed on NK cells, T cells, and some B cell subsets. The binding of CD226 on the surface of NK cells with ligands such as CD155 and CD112 on the surface of tumor cells can enhance the secretion of cytokines and cytotoxicity of NK cells. Studies have found that during aging and the initiation and progression of cancer, the expression of CD226 on NK cells gradually decreases, indicating that CD226 may mediate the immune surveillance function of NK cells. Additionally, CD226 plays an important role in maintaining the function of memory NK cells.

In this example, to investigate whether mbIL-21 expressed by NK cells affects the expression of CD226, the following method was employed: 1. 1E5-2E5 test cells were harvested and centrifuged at 200g-400g for 5 minutes at 4°C, after which the supernatant was discarded. 2. The cells were washed once with 200 μL of FACS Buffer (1% FBS in PBS), then centrifuged at 200g-400g for 5 minutes at 4°C, and the supernatant was discarded. 3. After adding 100 μL of directly conjugated anti-CD226 antibody (1:100), the cells were evenly dispersed by pipetting, and incubated at 4°C for 30-60 minutes. 4. After incubation, 200 μL/well of FACS Buffer was added, and the cells were centrifuged at 200g-400g for 5 minutes at 4°C, with the supernatant being discarded. 5. Step 4 was repeated twice. 6. After washing, the cells were resuspended in 200 μL/well of FACS Buffer and analyzed by flow cytometry.

The mbIL-21 used in this example was IL21SP-**IL21**-GGGGS-CD8 hinge-CD8TM.

The results are shown in Figure 7, which indicate that the expression of mbIL-21 on the surface of NK cells unexpectedly and significantly enhanced the expression of CD226 on the surface of NK cells.

**Discussion**

Cytokines such as IL-2 and IL-15 have multifaceted effects on immune cells, e.g., NK cells.

Although IL-2 can be used to prolong the persistence of NK cells in the host body, it can also lead to the massive proliferation of Treg cells that exert immunosuppressive effects. In clinical applications, it has been found that intravenous injection of IL-2 can cause symptoms such as fever and vomiting, and may also lead to disorders in water and salt metabolism, as well as functional abnormalities in the kidneys, liver, heart, and lungs, etc. The most common and severe complication is capillary leak syndrome, which often forces patients to discontinue treatment.

IL-15 shares some similar functions with IL-2, including stimulating the proliferation of activated T cells, generating cytotoxic effector T cells, and activating and maintaining NK cells. Unlike IL-2, IL-15 is generally not believed to cause massive proliferation of Treg cells. Additionally, IL-15 plays an important role in the generation and maintenance of memory NK cells. However, studies have found that prolonged exposure to IL-15 under long-term *in vitro* expansion conditions can lead to an exhausted phenotype in NK cells.

IL-21 activates the JAK-STAT, PI3K, and MAPK pathways. STAT3 plays a major role in the biological effects of IL-21, while STAT1 also contributes to IL-21-regulated gene expression. IL-21 has important roles in regulating the proliferation, cytotoxicity, memory formation and other aspects of NK cells. For example, IL-21 can enhance the secretion of IFN-γ by NK cells. Using IL-21-expressing K562 cells as feeder layers allows for the *in vitro* expansion of large numbers of highly activated NK cells and can mitigate the exhaustion that often occurs during NK cell culture.

Through research, the inventors unexpectedly discovered that by modifying IL-21 to be fixedly expressed on the surface of specific cells forming a membrane-bound IL-21 (mbIL-21), the *in vitro* expansion capacity of NK cells can be greatly enhanced, NK cell exhaustion can be reduced, and their *in vivo* lifespan in the host can be extended, thereby enhancing the cytotoxicity of NK cells against tumor cells. In particular, the optimized mbIL-21 protein can significantly promote NK cell proliferation and intracellular signaling, such as the phosphorylation of STAT3, offering broad clinical application prospects.

Furthermore, the mbIL-21 of the present invention can be used in combination with other factors such as IL-12, IL-15, and IL-18 to synergistically promote the generation of memory NK cells.

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

**Claims**

1. A recombinant protein, wherein the recombinant protein is a membrane-bound IL-21 (mbIL-21).

2. The recombinant protein of claim 1, wherein the membrane-bound IL-21 has the structure shown in Formula I:

$$Z1\text{-}Z2\text{-}Z3\text{-}Z4 \qquad (I)$$

wherein,

   Z1 is a signal peptide or absent;
   Z2 is an IL-21 polypeptide;
   Z3 is a CD8 hinge region;
   Z4 is a CD8 transmembrane region;
   "-" represents a peptide bond or a linker peptide.

3. The fusion protein of claim 2, wherein the membrane-bound IL-21 has a sequence shown in positions 1-249 or 25-249 of SEQ ID No: 1; or has a sequence shown in positions 1-246 or 22-246 of SEQ ID No: 2.

4. An isolated polynucleotide encoding the recombinant protein of claim 1.

5. A vector comprising the polynucleotide of claim 4.

6. A genetically engineered cell comprising the vector of claim 5, or having the polynucleotide of claim 4 integrated into its genome.

7. The genetically engineered cell of claim 6, wherein the genetically engineered cell is selected from the group consisting of: an embryonic stem cell, a mesenchymal stem cell, an iPSC cell (induced pluripotent stem cell), an NK cell, a T cell, a lymphocyte, and a combination thereof.

8. A pharmaceutical composition comprising the genetically engineered cell of claim 6 and a pharmaceutically acceptable carrier.

9. Use of the genetically engineered cell of claim 6 in the manufacture of a medicament for immunotherapy.

10. A cellular immunotherapy, which comprises the step of: administering a genetically engineered cell to a patient, wherein the genetically engineered cell comprises the vector of claim 5, or has the polynucleotide of claim 4 integrated into its genome.

Signal peptide

mbIL-21-CD8TM [ IL-21 | CD8 hinge | CD8 TM ]

Figure 1

Figure 2

Donor 0023     Donor z0276

mbIL21-CD8TM-mCherry     mbIL21-CD8TM-mCherry

R4: 38.978%     R4: 24.356%

mCherry

R5: 39.208%     R5: 25.101%

mbIL-21

Figure 3

Figure 4

Figure 5

Figure 6

Figure 7

## INTERNATIONAL SEARCH REPORT

| International application No. |
|---|
| **PCT/CN2023/111999** |

| A. | CLASSIFICATION OF SUBJECT MATTER |
|---|---|

C07K19/00(2006.01)i; C12N15/867(2006.01)i; C12N5/10(2006.01)i

According to International Patent Classification (IPC) or to both national classification and IPC

| B. | FIELDS SEARCHED |
|---|---|

Minimum documentation searched (classification system followed by classification symbols)

IPC:C07K,C12N

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, ISI of Web Science, 百度学术, BAIDU SCHOLAR, Patentics, 中国生物序列检索系统, China Biological Sequence Search System, NCBI Genbank, EBI, STNext: 申请人, applicant, 发明人, inventor, 连接, 连接子, CD8, GGGGS, GGGS, IL21, Interleukin21, TTTPA, CD-8, Interleukin-21, IL-21, 膜定位, signal peptide, Hinge, transmembrane, 信号肽, 铰链区, 跨膜区, 连接肽, linker.

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 109371062 A (BEIJING DINGCHENG TAIYUAN BIOTECHNOLOGY CO., LTD.) 22 February 2019 (2019-02-22)<br>    description, paragraphs 27-29 and SEQ ID No. 13 | 1-10 |
| A | CN 103243072 A (ZHEJIANG ZHONGYING LIFE ARK BIOTECHNOLOGICAL ENGINEERING CO., LTD.) 14 August 2013 (2013-08-14)<br>    entire document | 1-10 |
| A | CN 106754730 A (SHANGHAI BIOMED-UNION BIOTECHNOLOGY CO., LTD.) 31 May 2017 (2017-05-31)<br>    entire document | 1-10 |
| A | CN 111164100 A (AMGEN, INC.) 15 May 2020 (2020-05-15)<br>    entire document | 1-10 |
| A | US 2019255108 A1 (ICELL GENE THERAPEUTICS LLC) 22 August 2019 (2019-08-22)<br>    entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 October 2023** | **02 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/111999**

### C. DOCUMENTS CONSIDERED TO BE RELEVANT

| Category* | Citation of document, with indication, where appropriate, of the relevant passages | Relevant to claim No. |
|---|---|---|
| A | NITTO, C.D. et al. "Design and Characterization of Novel Antibody-Cytokine Fusion Proteins Based on Interleukin-21" *Antibodies*, Vol. vol. 11, 04 March 2022 (2022-03-04), pages 1-11 | 1-10 |

Form PCT/ISA/210 (second sheet) (July 2022)

## INTERNATIONAL SEARCH REPORT

International application No.

**PCT/CN2023/111999**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2023/111999**

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **10**
    because they relate to subject matter not required to be searched by this Authority, namely:

    Claim 10 relates to cellular immunotherapy, which falls within subject matter for which no search is performed (PCT Rule 39.1(iv)). However, a search is still carried out on the basis of a corresponding pharmaceutical use thereof.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2023/111999**

| Patent document cited in search report | | | Publication date (day/month/year) | Patent family member(s) | | | Publication date (day/month/year) |
|---|---|---|---|---|---|---|---|
| CN | 109371062 | A | 22 February 2019 | None | | | |
| CN | 103243072 | A | 14 August 2013 | None | | | |
| CN | 106754730 | A | 31 May 2017 | None | | | |
| CN | 111164100 | A | 15 May 2020 | BR | 112020002013 | A2 | 28 July 2020 |
| | | | | BR | 112020002013 | B1 | 24 January 2023 |
| | | | | WO | 2019028316 | A1 | 07 February 2019 |
| | | | | CO | 2020001113 | A2 | 15 May 2020 |
| | | | | HRP | 20220404 | T1 | 27 May 2022 |
| | | | | UY | 37829 | A | 31 January 2019 |
| | | | | CR | 20200099 | A | 24 July 2020 |
| | | | | PH | 12020500231 | A1 | 11 January 2021 |
| | | | | EP | 4029877 | A1 | 20 July 2022 |
| | | | | TN | 2020000015 | A1 | 04 October 2021 |
| | | | | MA | 56289 | A | 10 June 2020 |
| | | | | MA | 56289 | B1 | 29 April 2022 |
| | | | | EP | 3661954 | A1 | 10 June 2020 |
| | | | | EP | 3661954 | B1 | 09 February 2022 |
| | | | | ES | 2910969 | T3 | 17 May 2022 |
| | | | | SG | 11202000821 | SA | 27 February 2020 |
| | | | | PT | 3661954 | T | 14 April 2022 |
| | | | | TW | 201920239 | A | 01 June 2019 |
| | | | | TWI | 798245 | B | 11 April 2023 |
| | | | | RS | 63101 | B1 | 29 April 2022 |
| | | | | HUE | 058233 | T2 | 28 July 2022 |
| | | | | TW | 202246308 | A | 01 December 2022 |
| | | | | US | 2019046611 | A1 | 14 February 2019 |
| | | | | US | 11541103 | B2 | 03 January 2023 |
| | | | | CA | 3071376 | A1 | 07 February 2019 |
| | | | | JOP | 20200020 | A1 | 02 February 2020 |
| | | | | CL | 2020000252 | A1 | 21 August 2020 |
| | | | | MY | 195974 | A | 27 February 2023 |
| | | | | JP | 2022116099 | A | 09 August 2022 |
| | | | | KR | 20220092652 | A | 01 July 2022 |
| | | | | LT | 3661954 | T | 11 April 2022 |
| | | | | AU | 2022228122 | A1 | 29 September 2022 |
| | | | | BR | 122021015266 | B1 | 24 January 2023 |
| | | | | IL | 272137 | A | 31 March 2020 |
| | | | | JP | 2019033743 | A | 07 March 2019 |
| | | | | JP | 7079171 | B2 | 01 June 2022 |
| | | | | AU | 2018311079 | A1 | 13 February 2020 |
| | | | | AU | 2018311079 | B2 | 23 June 2022 |
| | | | | PL | 3661954 | T3 | 16 May 2022 |
| | | | | SI | 3661954 | T1 | 31 May 2022 |
| | | | | KR | 20200035291 | A | 02 April 2020 |
| | | | | KR | 102414120 | B1 | 28 June 2022 |
| | | | | DK | 3661954 | T3 | 19 April 2022 |
| | | | | MX | 2020001328 | A | 20 March 2020 |
| | | | | SA | 520411230 | B1 | 08 November 2022 |
| | | | | CL | 2022000300 | A1 | 23 September 2022 |
| US | 2019255108 | A1 | 22 August 2019 | US | 11173179 | B2 | 16 November 2021 |

Form PCT/ISA/210 (patent family annex) (July 2022)

## INTERNATIONAL SEARCH REPORT
### Information on patent family members

International application No.

**PCT/CN2023/111999**

| Patent document cited in search report | Publication date (day/month/year) | Patent family member(s) | Publication date (day/month/year) |
|---|---|---|---|
| | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning: A Laboratory Manual. Cold Spring Harbor Laboratory Press, 1989